# EUROPEAN PATENT SPECIFICATION

(11) **EP 2 223 656 B1**
(45) Date of publication and mention of the grant of the patent: **31.12.2014**
(21) Application number: 09164082.1
(22) Date of filing: 30.06.2009
(51) Int. Cl.: A61B 10/00, B01L 3/00, B01L 3/14

(54) **Enhanced test tube for collecting, transporting and extracting faeces samples**
Verbessertes Reagenzglas zum Sammeln, Transportieren und Entnehmen von Fäzesproben
Tube de test amélioré pour collecter, transporter et extraire des échantillons fécaux

(30) Priority: 25.02.2009 IT MI20090265
(43) Date of publication of application: 01.09.2010
(73) Proprietor: Sentinel CH S.p.A., 20152 Milano (IT)
(72) Inventor: De Luca, Ugo, 20144, MILANO (IT); Roveda, Luigi, 20151, MILANO (IT)
(74) Representative: Borsano, Corrado

(56) References cited:
- EP-A- 1 366 715
- EP-A- 1 986 006
- DE-B3-102007 057 760

## Description

The present invention relates to an extracting and sampling device for collecting faeces samples, particularly suitable for performing laboratory diagnostic tests in a total laboratory automation, in particular for dosing one or more analytes, such as for example hemoglobin, Helicobacter pylori and the like, and allowing the screening of markers of diagnostic interest for purposes of prevention and treatment.

In fact, such a faeces sampling device may be received and processed by robotic systems in highly automated analysis laboratories as the samples of body fluids (whole blood, plasma, serum, etc.) are processed. These systems may implement the following pre-analytical steps on the sampling device, as required: identifying the sample by reading a bar code, de-capping (removing the test tube cap) or piercing (perforating the closure film of the test tube for the subsequent sampling by means of a specific probe), adding an appropriate extraction buffer, dissolving/dispersing the taken dry sample, centrifuging, transferring to the analysis station, hermetically sealing by means of a film and storing in specific, controlled temperature lockers for further analysis.

It is known from Patent Application EP 1366715 to Sentinel CH. S.p.A. to employ an extracting and sampling test tube for collecting faeces samples which may be directly used on automated analysis systems.

Such a test tube is practical for the patient who has a simple and practical collecting device, and easy to be used by the laboratory technician as it may be directly employed with several commercially available automated analysis systems, e.g. the automatic analyzers for immunological tests and clinical chemistry.

Such a test tube for collecting faeces samples comprises an internally hollow container body which is open at the two ends.

At a first end, the container body is equipped with a cap provided with a threaded stick for collecting faeces samples, said threaded stick axially protruding into the container body when the cap is applied to the first end of the container body.

Furthermore, such a test tube comprises a partition in an intermediate position within said container body for separating an upper compartment from a lower compartment within the container body. Such a partition has an axial hole so as to allow the threaded stick to be introduced so as to retain the exceeding faeces in the upper compartment, and to permit the threaded zone of the stick to be introduced into said lower compartment.

The body container is then opened at the second end, adapted to receive an extraction buffer solution, and is provided with a second cap removably applicable to the container body, so that said extracting test tube may be directly used as a primary sampling test tube to be fitted on a sample-holder plate of an automatic analyzer.

In order to achieve this result consisting in being able to directly conduct the sample analysis on an automatic analyzer, placing the container body in a housing of the automatic analyzer with the first end cap facing downwards and removing the second cap is needed. In the body container zone closed by the second cap, a buffer solution is indeed contained or may be inserted at the laboratory.

The user who should collect the faeces sample, unscrews the first cap connected to the stick and sinks the stick in the faeces, thus collecting the sample. Once the faeces sample has been dispersed in the buffer solution, possibly already present in the test tube or subsequently added, the test tube itself may be used as a primary sampling test tube to be directly fitted on the sample-holder plate of an automatic analyzer.

Only at this point, the second cap is removed so as to allow the automatic analyzer probe to take an aliquot of the buffer solution, containing the dissolved/dispersed faeces sample, on which the analyte object of the research will be dosed.

Further examples of known test-tubes are provided in DE 10 2007 057760 which discloses a sampling instrument for human stool having a retention chamber for surplus sample material and a disposal chamber to dissolve the sample in a fluid, the instrument being provided with a dispenser element at the opposite end of the tube respect to the sample extractor, and in EP 1 986 006 which discloses a feces collecting container provided with an holding part and a fecal sampling part, a closing cap being provided at the second end of the test tube, opposite to the end in which the holding part is inserted.

The test tube of known type and shown heretofore has however some drawbacks. A first drawback consists in that the test tube of known type has no grip to be handled by the robotic systems sorting the sampling devices (taking test tubes) to the various automatic analyzers once the expected pre-analytical steps have been carried out. This results in these steps being completed by the manual intervention of the laboratory technician.

A further improvable aspect is that the second cap closing the zone of the test tube containing or which may then be fed with the buffer solution could be accidentally removed by the user, thus causing the dispersion of the buffer solution and therefore the non-availability of the test tube itself, which should be eliminated with the need of repeating the sampling with a new test tube. In fact, the user could get confused during the operations of sample collection and accidentally unscrew the wrong cap.

Furthermore, the laboratory technician who receives the test tube containing the sample to be analyzed has no way to realize if such a cap has been opened and then closed, and therefore if part of the buffer solution has gone dispersed upon handling the test tube by the user. A part of the buffer solution could then be gone dispersed or it may have been somehow contaminated and the laboratory technician would have no way of realizing it.

Therefore, the primary task of the present invention is to solve the drawbacks that affect the collecting and sampling systems of the known type.

Within this task, the object of the present invention is thus to provide a test tube for extracting and collecting faeces samples, suitable for being received and processed by the robotic systems in the highly automated analysis laboratories as the samples of body fluids (whole blood, plasma, serum, etc.) are processed. These systems may implement on the sampling device the pre-analytical steps needed for the overall automation of the process: identifying the sample by reading a bar code, centrifuging, de-capping or piercing, adding an appropriate extraction buffer and dissolving the taken dry sample, transferring to the analysis station, hermetically sealing by means of a film and possible storing in specific controlled temperature lockers for further analysis.

Again, it is the object of the present invention to provide an extracting and sampling test tube which allows the faeces samples to be collected, both directly in the extraction buffer (existing in the device compartment intended therefor) and in dry conditions, the latter being particularly suitable for collecting and shipping biological origin materials by mail and the like.

It is a further object of the present invention to provide an enhanced test tube for extracting and collecting faeces samples which allows the laboratory technician to effectively and immediately identify possible tampering of the test tube zone containing the buffer solution if the test tube is provided to the user when it already contains the buffer solution.

It is a further object of the present invention to provide a test tube for extracting and collecting faeces samples that is provided with a cap which, in addition to the ability of effectively and immediately highlighting possible tampering, may be perforated by a specific-purpose probe existing on the automatic analyzer or processing module of the pre-analytical steps so as to avoid the operations of manually or non-automatically opening the individual test tubes by the technician.

These objects are achieved by a sample test tube according to what indicated in claim 1 and in its dependent claims.

Further features of the invention will be more apparent from the following detailed description, which refers to a merely exemplary embodiment thus not limiting the same, illustrated in the accompanying drawings, in which:
figure 1 shows a sectional front view of an extracting and collecting test tube of the type known from the state of art;
figure 2 shows a diagrammatic view of the container body of the test tube according to the present invention;
figure 3 shows a detail of the sample collecting device of the test tube according to the present invention;
figure 4 shows a detail of the test tube according to the present invention where the sample collecting device is shown to be inserted into the container body of said test tube;
figure 5 shows a sectional assembly view of the test tube according to the present invention;
figure 6 shows a sectional detail of the lower cap of the test tube in figure 5;
figures 7A and 7B show two views of a closure cap for one of the ends of the test tube according to the present invention;
figures 8A and 8B show two views of a variant of the closure cap in figures 7A and 7B.

The extracting test tube for collecting faeces samples according to the invention, indicated by numeral 1 as a whole, will now be described with the aid of the figures.

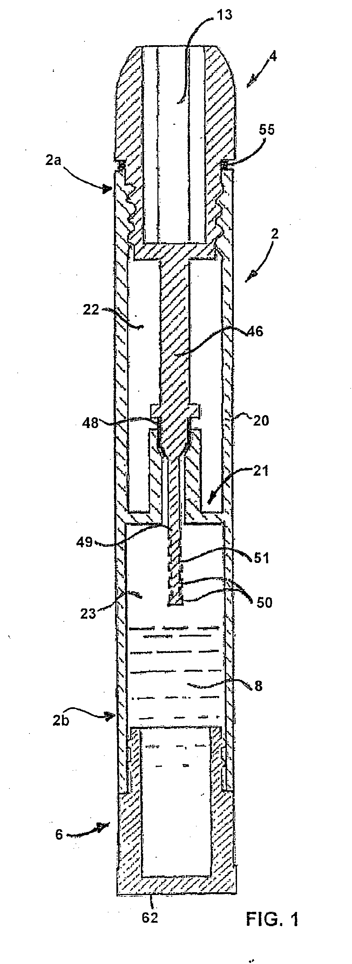

Specifically, Figure 1 depicts the sample collecting test tube comprising a lower cap 6 of the type known from the state of the art.

The test tube comprises a container body 2, a first cap 4 at a first end 2a of the container body 2. In the case of a test tube of the known type, a second cap 6 is provided at the second end 2b of the test tube, which is substantially cylindrical and suitable for pressure-fitting within the end 2b of the container body 2.

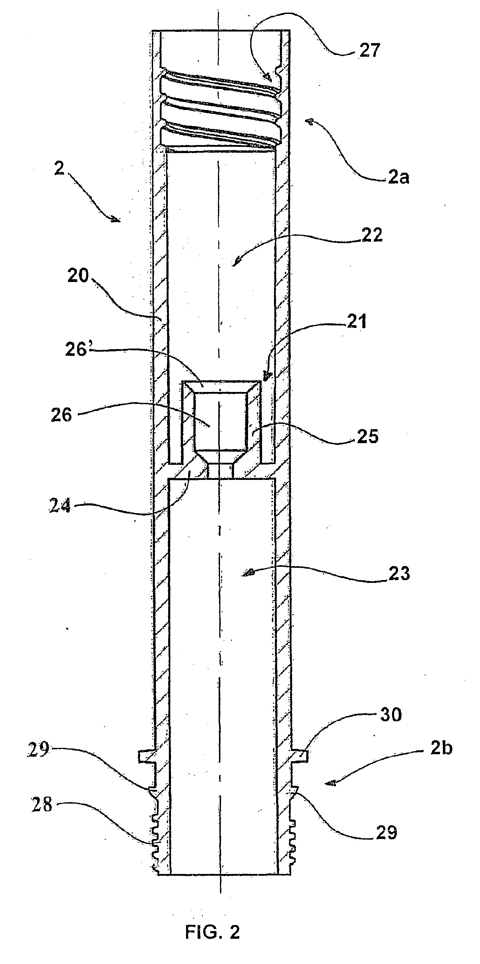

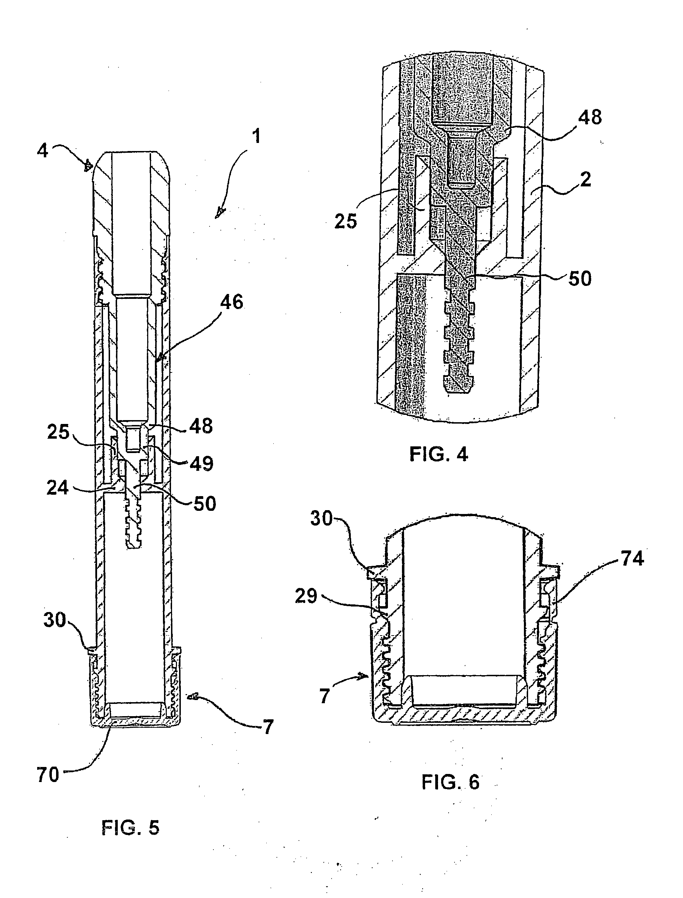

Instead, figure 2 shows, again in section view, just the container body 2 of the sample collecting test tube according to the present invention, from which the other members are disassembled. The test tube of the present invention may be seen as assembled in the section of figure 5.

The container body 2 comprises a substantially cylindrical body 20 which is internally hollow and open at the two ends end 2a and 2b. Within the cylindrical body 20, a partition 21 is provided in an intermediate position, which divides the interior of the container body 2 into a first compartment 22 and a second compartment 23.

The partition 21 comprises an annular flange 24 radially projecting towards the interior of the cylindrical container. A cylindrical ridge 25 axially protrudes from the side of the annular flange 24 facing towards the first compartment 22. The partition 21 has an axial through hole 26 which axially passes through the cylindrical ridge 25 and the annular flange 24. The hole 26 has at the top a conical-profiled, substantially tapered zone 26' with an increasing diameter as compared to the lower zone.

An internal thread 27 is provided in the internal surface of the cylindrical body 20, within the end 2a of the first compartment 22.

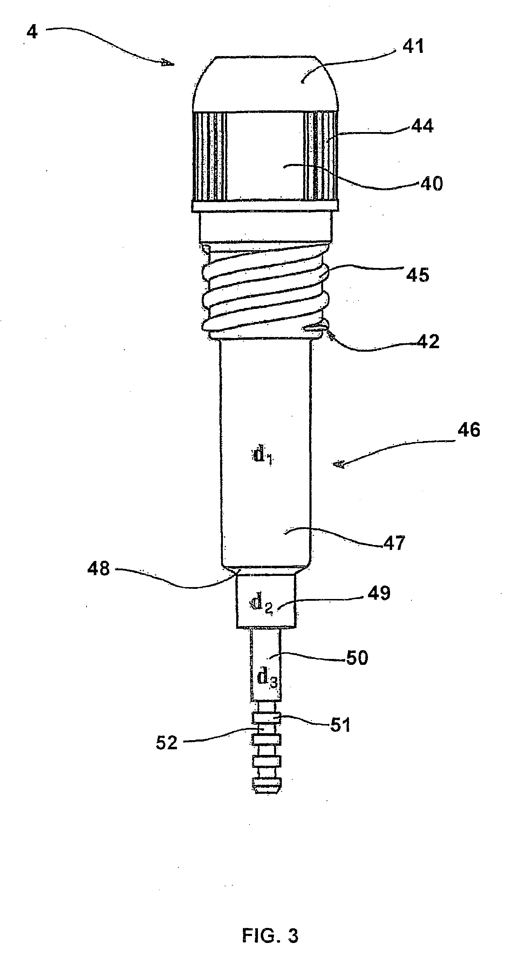

As seen in figure 3, the first cap 4 according to the present invention comprises a body consisting of a substantially cylindrical middle zone 40 with an external diameter substantially equal to the external diameter of the cylindrical body 20, a dome-shaped upper zone 41 placed above the middle zone, and a substantially cylindrical lower zone 42 provided with an appropriate thread 45 adapted to be fitted in the corresponding thread 27 provided within the end 2a of container body 2.

A knurl 44 consisting of a plurality of ribs and grooves is provided on the external surface of the middle zone 40 of the body of the upper cap 4.

A shank or stick 46, presenting a first substantially cylindrical section 47 with a diameter d₁ smaller than the diameter of the lower zone 42 of the cap 4, axially protrudes from the lower zone 42 of the first cap 4.

Then, the stick 46 further tapers towards the end and presents a second portion 49 with a diameter d₂, smaller than d₁, with a conical joint 48 between the two cylindrical sections 47 and 49. Finally, a third cylindrical section 50 with an even lower external diameter d₃ originates with an abrupt change in diameter and free from joints from the second portion 49 with a diameter d₂.

As seen from figure 5 showing the test tube when assembled, the conical joint zone 48 between the sections 47 and 49 has a profile suitable for matching the conical-profiled tapered zone 26' obtained on the partition 21 of the container body.

The third cylindrical section 50 of the stick 46 comprises, at its free end zone, a thread consisting of a plurality of annular collars 51 spaced from one another so as to form as many annular spaces 52 between said collars 51.

According to the present invention, at the end 2b of the container body 2 opposite to the first end 2a with which said first cap 4 with the sampling stick 46 is associated, there are provided specific gripping means for automatic machines that allow the totally automated handling of the test tube.

Specifically, according to the embodiment illustrated by way of example in figures 2, 5 and 6, these gripping means consist of an annular profile 30 radially protruding outwards and adapted to be a striker for the grip by robotic arms adapted to automatically handle the test tube itself.

According to the present invention, tamperproof closure means may advantageously be provided at the second end 2b of the test tube, the one facing the end 2a on which the first cap 4 is provided.

According to a first embodiment illustrated in figures 5 and 6, these tamperproof closure means may comprise a second cap 7 which comprises a cylindrical body having an internal diameter substantially equal to the external diameter of the container body 2, said second cap 7 having an internal thread 71 adapted to be screwed at the terminal end of said container body 2, said terminal end of said container body 2 having in turn an external counter-thread 28 suitable for engaging said thread 71.

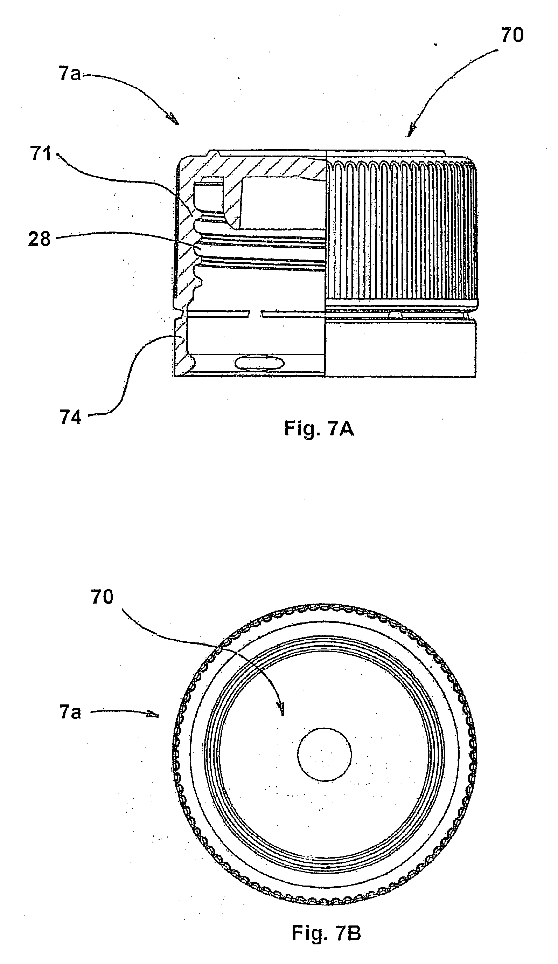

Two possible embodiments of the second cap 7 are shown hereinafter. A first embodiment of the second cap is indicated by 7a and it is shown in figures 7A and 7B.

According to a first embodiment shown in figures 7A and 7B, the second cap 7a is closed at its central portion 70. In this case, it will be necessary to unscrew the cap in order to open the test tube, which operation may also be carried out in an automatic manner by the analysis machines suitable for carrying out the de-capping of the test tubes.

Again with reference to the figures, the second cap 7a may advantageously be provided with safety means 74 which prevent the accidental unscrewing thereof.

Specifically, according to the preferred embodiment shown by way of example in figures 2, 6 and 7A, these safety means may comprise an annular member 74 suitable for interacting with a specific striker 29 provided on the external surface of the container body 2 by means of a specific internal groove adapted to receive said striker 29.

A knurl, seen for example in figure 7A and consisting of a plurality of ribs and grooves, may advantageously be provided on the external surface of the second cap 7a.

Said annular member 74 is connected to the body of the second cap 7a in an unmatchable manner by means of one or more connecting members not depicted in the figures.

Said one or more connecting members may consist of peduncles or tongues, for example, made of a material preferably consisting of the same plastic material as the second cap 7a and the annular member 74 itself. These peduncles retain said safety annular member 74 integral with the second cap 7a as long as it is completely screwed on said container body 2.

The safety annular member 74 operates as follows.

When the second cap 7a is completely screwed to the container body 2, the connecting members or peduncles (not depicted in the figures) connecting the cylindrical body of said second cap 7a to said annular member 74 prevent the cap 7a itself from accidentally open. In fact, the annular ring 74 prevents the cap 7a from unscrewing off the container body 2 and prevents the cap 7a itself from rotating and therefore unscrewing by striking against the striker 29 provided on said container body 2.

In fact, the rotation of the cap 7a in the unscrewing direction also causes an axial translation of the cap 7a in the direction along which the cap moves away from the container body, because of the presence of the helical thread. The member 74, which is dragged by the presence of the connecting members or peduncles in the roto-translation of the cap 7a, prevents the cap itself from unscrewing since, striking against the striking member 29 integral with the container body 2, it prevents the translating movement of the cap 7a in an axial direction towards the direction along which the cap 7a moves away from the container, thus ultimately preventing the cap itself from unscrewing.

In order to unscrew the cap, a sufficient twisting torque need to be exerted on the cap 7a in the unscrewing direction so as to break said connecting members or peduncles, such an unscrewing operation may be manually performed by the laboratory technician or by an automatic machine.

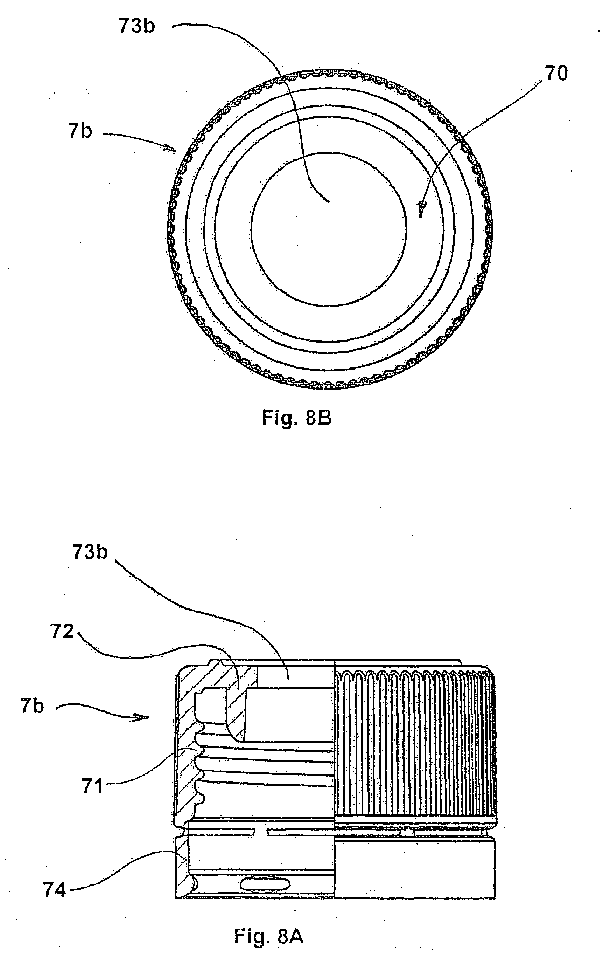

According to an alternative embodiment, the second cap 7 may be provided as shown in figures 8A and 8B, where it is indicated by the reference numeral 7b. In this case, the tamperproof closure means placed close to the test tube end 2b comprise a second cap 7b having, in an axial position on the bottom surface 70, a through hole 73b hermetically sealed by a membrane or film made of polymeric material, e.g. aluminium-polythene.

Thereby, it is possible for the automatic machine to take the buffer solution by means of a film piercing and picking up operation instead of preliminary unscrewing the cap itself, for example.

According to the first embodiment of the test tube according to the present invention, a polymeric material film suitable for being perforated during the piercing operation may be directly applied to the second end 2b of the container body 2.

In each of these cases, the compartment 23 may comprise the buffer solution or may be dry. In all the shown examples, a test tube may be easily opened at one end only, the one closed by the first cap 4, whereas the described safety means are provided at the opposite end, at the second cap 7, with the major advantage of avoiding the patient from opening the wrong cap with the relating problems.

In the second embodiment in which the second cap 7b is provided with a through hole closed by a film, the second cap 7b may advantageously be welded directly to the container body 2 by a welding operation by means of an ultrasonic method or similar known methods. By means of this further measure, the further benefit of preventing the user from accidentally opening the second cap 7b may be obtained, thus achieving an even greater safety.

Therefore, it has been shown how the sampling test tube according to the present invention achieves the task and the intended objects.

Specifically, it has been disclosed how the test tube according to the present invention is suitable for being directly placed into the sample-holder housing of an automatic machine with the end 2b, at which said gripping means 30 are provided, facing upwards. In order to be able to transfer, place or remove said test tube in an automatic manner, so as to further reduce the interventions by the laboratory technician, the test tube offers a striker member to the robotic arm or however to the gripping member provided on said automatic machine.

Thereby, the sampling test tube may be received and processed by the robotic systems in the highly automated analysis laboratories as the samples of body fluids are processed.

The possibility that the sampling test tube according to the present invention is handled in an automatic manner by the automated analysis systems allows to obtain several advantages in terms of process automation according to the above. Again, another advantage may be obtained if the test tube comprises tamperproof closure means suitable for closing the second end 2b of the container body 2, where said tamperproof closure means comprise a second cap 7. In this case, the robotic systems may perform the de-capping (i.e. providing for the removal of the cap) by also automating this step, and if the user is provided with the test tube when it already contains the buffer solution, the handling by an automatic machinery ensures that the test tube is moved without the risk of spilling the buffer solution contained in the test tube because of an error by the technician.

The striker 29 allows the grip by the handler for transferring the test tube to the stations where the second cap 7a is unscrewed or the film is pierced in the case of a cap 7b provided with a through hole closed by a film or in the case of a film directly applied to the end 2b of the container body 2, thus allowing a full automation of the operations before the analysis.

The buffer solution, generally indicated by reference numeral 8 in figure 1, may be a solution with a pH suitable for storing the sample containing specific stabilizers, for example. The user who should collect a faeces sample, unscrews the upper cap 4 and takes it from the faeces by means of the stick 46. Therefore, by extracting the stick 46 from the faeces, the faeces samples are retained in the annular spaces 52 made on the stick 46 and the exceeding faeces also remain about the stick 46.

Therefore, the collection of the sample may take place in complete safety both if the test tube is already provided to the user when it contains the buffer solution, and if said solution is subsequently added at the laboratory. In this second case, the sample is collected completely dry, thus allowing the test tube itself to be sent by mail, which is forbidden by the laws in force in many countries if the device contains a liquid, and the buffer solution may then be added at the laboratory manually or automatically by means of specific automated systems.

Therefore, the second cap 7 is removed and the extracting test tube is placed in a specific housing of an automatic analyzer in order to perform the analysis on an aliquot of the buffer solution containing the faeces samples. In order to obtain this result, the external diameter of the container body 2 will be advantageously designed so as to match the size of the test tube-holder housings of the automatic analyzers.

Therefore, it has been shown how, due to the test tube according to the present invention, the handling of the test tube may be completely automated, thus allowing the use of a robotic system that, in addition to the pre-analytical steps, also performs the steps of placing/loading the test tube in the test tube-holder housing of the automatic analyzer, unloading the test tube from the tube-holder plate, possibly closing by means of a film and storing in refrigerated lockers for possible analysis repetitions.

Furthermore, the preferred embodiments of the test tube according to the present invention, comprise tamperproof closure means, such as the warranty collar 74 which is detached from the closure cap during the opening, thus allowing the laboratory technician to easily verify if the part of the test tube containing the buffer solution or however containing the end of the stick 46 for collecting the samples has been accidentally opened.

In the case of the presence of the warranty collar 74, the striker ring 30 also plays the role of striker for such a collar so that, once the collar 74 is detached from the cap 7 to open the same, said collar may not slip under gravity along the test tube.

Either if it is used a second cap with a safety device, or it is used a film made of a pierceable material directly applied to the test tube, the further object to make the closure of the second end 2b of the test tube error-proof for the user is achieved, thus making possible tampering of such a closure immediately identifiable by the laboratory technician.

This aspect makes the collecting system safer and more error-proof both if the test tube is supplied when it already contains the buffer solution, and if it is provided without a buffer solution, in this case the sample being stored when dry.

Several modifications and detail variations within the reach of a person skilled in the art may be made to the present embodiment of the invention, without however departing from the scope of protection of the invention as set forth in the appended claims.

## Claims

1. A sampling test tube (1) for collecting faeces samples of the type comprising:
- a container body (2), internally hollow and open at both ends (2a, 2b), adapted to receive a buffer solution,
- a first cap (4) provided with a threaded stick (46) for collecting faeces samples, said threaded stick axially protruding within the container body, when said first cap (4) is applied to a first end (2a) of the container body (2),
- a partition (21) provided in an intermediate position within said container body (2) in order to separate a first compartment (22) from a second compartment (23) within said container body;
**characterized in that** it further comprises gripping means (30) integral with the second end (2b) of said container body (2) facing said first end (2a) and suitable for forming a gripping striker for the automatic analysis machines that may therefore process the test tube in an automated manner, and **in that** it further comprises tamperproof closure means (7, 74, 29) adapted to safely close said second end (2b) of said container body (2), said tamperproof means for closing the second end (2b) of said container body (2) comprise a film suitable for being perforated by a probe of the type with which an automatic analyzer or an automatic processing module of the pre-analytical steps is provided.

2. A sampling test tube (1) according to claim 1, **characterized in that** said gripping means (30) comprise an annular profile radially projecting outwards with respect to said container body (2) and suitable for forming a striker for the grip by the automatic processing module of the pre-analytical steps.

3. A sampling test tube (1) according to any one of the preceding claims, **characterized in that** said gripping means are placed at the second end (2b) of the test tube opposite to the first end (2a) to which said cap (4) is applied.

4. A sampling test tube (1) according to the preceding claim, **characterized in that** said tamperproof closure means comprise a second cap (7) which comprises a bottom surface (70) and which further has an internal thread (71) suitable for allowing said second cap (7a) to be screwed to the second end (2b) of said container body (2), said second end (2b) of said container body (2) having in turn an external counter-thread (28) suitable for engaging said thread (71) of the cap (7).

5. A sampling test tube (1) according to claim 4, **characterized in that** said tamperproof closure means further comprise an annular member (74) suitable for interacting against a specific striker (29) provided on the external surface of the container body (2) by means of a specific internal groove adapted to receive said striker (29), said annular member (74) being connected to said second cap (7a) by means of one or more connecting members in an unmatchable manner.

6. A sampling test tube (1) according to the preceding claim, **characterized in that** said one or more connecting members consist of tongues or peduncles made of the same plastic material as said second cap (7a) and said annular member (74).

7. A sampling test tube (1) according to one or more of the preceding claims, **characterized in that** said second cap (7b) comprises a bottom surface (70) provided with a through hole (73b).

8. A sampling test tube (1) according to claim 7, **characterized in that** said through hole (73b) is closed by a film suitable for being perforated by a probe of the type with which an automatic analyzer or an automatic processing module of the pre-analytical steps is provided.

9. A sampling test tube (1) according to claim 8, **characterized in that** said film is made of aluminium-polythene.

10. A sampling test tube (1) according to claim 7, **characterized in that** said second cap (7b) is welded to said container body (2).

11. A sampling test tube (1) according to claim 1, **characterized in that** said film is made of aluminium-polythene or the like.

## Patentansprüche

1. Probennahmeröhrchen (1) zum Sammeln von Fäkalproben von dem Typ, der umfasst:
- einen Behälterkorpus (2), der innen hohl und an beiden Enden (2a, 2b) offen ist und ausgebildet ist, um eine Pufferlösung aufzunehmen,
- eine erste Kappe (4), die mit einem Gewindestab (46) zum Sammeln von Fäkalproben versehen ist, wobei der Gewindestab axial innerhalb des Behälterkorpus vorsteht, wenn die erste Kappe (4) an einem ersten Ende (2a) des Behälterkorpus (2) aufgebracht ist,
- eine Trennwand (21), die in einer Zwischenposition innerhalb des Behälterkorpus (2) vorgesehen ist, um einen ersten Raum (22) von einem zweiten Raum (23) innerhalb des Behälterkorpus zu trennen;
**dadurch gekennzeichnet, dass** es ferner umfasst, Greifmittel (30), die einstückig mit dem zweiten Ende (2b) des Behälterkorpus (2) sind, dem ersten Ende (2a) zugewandt sind und geeignet sind, um einen Greifanschlag für die automatischen Analysemaschinen zu bilden, die daher das Proberöhrchen in einer automatisierten Weise verarbeiten können, und dass es ferner manipulationssichere Verschlussmittel (7, 74, 29) umfasst, die ausgebildet sind, um das zweite Ende (2b) des Behälterkorpus (2) sicher zu verschließen, wobei die manipulationssicheren Mittel zum Verschließen des zweiten Endes (2b) des Behälterkorpus (2) eine Folie umfassen, die geeignet ist, um durch eine Sonde von dem Typ, mit dem eine automatische Analysevorrichtung oder ein automatisches Verarbeitungsmodul der Voranalyseschritte versehen ist, Typ perforiert zu werden.

2. Probennahmeröhrchen (1) nach Anspruch 1, **dadurch gekennzeichnet, dass** die Greifmittel (30) ein ringförmiges Profil umfassen, das mit Bezug auf den Behälterkorpus (2) radial vorsteht und geeignet ist, einen Anschlag für den Griff durch das automatische Verarbeitungsmodul der Voranalyseschritte zu bilden.

3. Probennahmeröhrchen (1) nach einem der vorhergehenden Ansprüche, **dadurch gekennzeichnet, dass** die Greifmittel an dem zweiten Ende (2b) des Proberöhrchens entgegengesetzt zu dem ersten Ende (2a), an dem die Kappe (4) aufgebracht ist, angeordnet sind.

4. Probennahmeröhrchen (1) nach dem vorhergehenden Anspruch, **dadurch gekennzeichnet, dass** die manipulationssicheren Verschlussmittel eine zweite Kappe (7) umfassen, die eine untere Fläche (70) umfasst, und die ferner ein Innengewinde (71) aufweist, das dafür geeignet ist, zuzulassen, dass die zweite Kappe (7a) an das zweite Ende (2b) des Behälterkorpus (2) geschraubt werden kann, wobei das zweite Ende (2b) des Behälterkorpus (2) wiederum ein äußeres Gegengewinde (28) aufweist, das für den Eingriff des Gewindes (71) der Kappe (7) geeignet ist.

5. Probennahmeröhrchen (1) nach Anspruch 4, **dadurch gekennzeichnet, dass** die manipulationssicheren Verschlussmittel ferner ein ringförmiges Element (74) umfassen, das zur Wechselwirkung gegen einen spezifischen Anschlag (29), der an der Außenfläche des Behälterkorpus (2) vorgesehen ist, mittels einer spezifischen inneren Nut geeignet ist, die ausgebildet ist, um den Anschlag (29) aufzunehmen, wobei das ringförmige Element (74) mit der zweiten Kappe (7a) mittels eines oder mehrerer Verbindungselemente auf eine vereinzelbare Weise verbunden ist.

6. Probennahmeröhrchen (1) nach dem vorhergehenden Anspruch, **dadurch gekennzeichnet, dass** das eine oder die mehreren Verbindungselemente aus Zungen oder Stängeln bestehen, die aus dem gleichen Kunststoffmaterial wie die zweite Kappe (7a) und das ringförmige Element (74) gebildet sind.

7. Probennahmeröhrchen (1) nach einem oder mehreren der vorhergehenden Ansprüche, **dadurch gekennzeichnet, dass** die zweite Kappe (7b) eine untere Fläche (70) umfasst, die mit einem Durchgangsloch (73b) versehen ist.

8. Probennahmeröhrchen (1) nach Anspruch 7, **dadurch gekennzeichnet, dass** das Durchgangsloch (73b) mit einer Folie verschlossen ist, die dafür geeignet ist, durch eine Sonde von dem Typ, mit dem eine automatische Analysevorrichtung oder ein automatisches Verarbeitungsmodul der Voranalyseschritte versehen ist, perforiert zu werden.

9. Probennahmeröhrchen (1) nach Anspruch 8, **dadurch gekennzeichnet, dass** die Folie aus Aluminium-Polythen hergestellt ist.

10. Probennahmeröhrchen (1) nach Anspruch 7, **dadurch gekennzeichnet, dass** die zweite Kappe (7b) an den Behälterkorpus (2) geschweißt ist.

11. Probennahmeröhrchen (1) nach Anspruch 1, **dadurch gekennzeichnet, dass** die Folie aus Aluminium-Polythen oder dergleichen hergestellt ist.

## Revendications

1. Tube de test d'échantillonnage (1) pour collecter des échantillons fécaux du type comprenant :
- un corps formant récipient (2), creux à l'intérieur et ouvert aux deux extrémités (2a, 2b) adapté pour recevoir une solution tampon,
- un premier capuchon (4) doté d'une tige filetée (46) pour collecter des échantillons fécaux, ladite tige filetée faisant saillie axialement à l'intérieur du corps formant récipient, lorsque ledit premier capuchon (4) est appliqué à une première extrémité (2a) du corps formant récipient (2),
- une cloison (21) fournie dans une position intermédiaire à l'intérieur dudit corps formant récipient (2) afin de séparer un premier compartiment (22) d'un second compartiment (23) à l'intérieur dudit corps formant récipient ;
**caractérisé en ce qu'**il comprend en outre des moyens de préhension (30) solidaires de la seconde extrémité (2b) dudit corps formant récipient (2) faisant face à ladite première extrémité (2a) et appropriés pour former un percuteur de préhension pour les machines d'analyse automatique qui peuvent ainsi traiter le tube de test d'une manière automatisée, et **en ce qu'**il comprend en outre des moyens de fermeture inviolables (7, 74, 29) adaptés pour fermer de manière sûre ladite seconde extrémité (2b) dudit corps formant récipient (2), lesdits moyens inviolables destinés à fermer la seconde extrémité (2b) dudit corps formant récipient (2) comprennent un film approprié pour être perforé par une sonde du type avec laquelle un analyseur automatique ou un module de traitement automatique des étapes pré-analytiques est fourni.

2. Tube de test d'échantillonnage (1) selon la revendication 1, **caractérisé en ce que** lesdits moyens de préhension (30) comprennent un profil annulaire faisant saillie radialement vers l'extérieur par rapport audit corps formant récipient (2) et approprié pour former un percuteur pour permettre la préhension par un module de traitement automatique des étapes pré-analytiques.

3. Tube de test d'échantillonnage (1) selon l'une quelconque des revendications précédentes, **caractérisé en ce que** lesdits moyens de préhension sont placés au niveau de la seconde extrémité (2b) du tube de test à l'opposé de la première extrémité (2a) à laquelle ledit capuchon (4) est appliqué.

4. Tube de test d'échantillonnage (1) selon la revendication précédente, **caractérisé en ce que** lesdits moyens de fermeture inviolables comprennent un second capuchon (7) qui comprend une surface inférieure (70) et qui possède en outre un filetage interne (71) approprié pour permettre audit second capuchon (7a) d'être vissé sur la seconde extrémité (2b) dudit corps formant récipient (2), ladite seconde extrémité (2b) dudit corps formant récipient (2) ayant à son tour un contre-filetage externe (28) approprié pour se mettre en prise avec ledit filetage (71) du capuchon (7).

5. Tube de test d'échantillonnage (1) selon la revendication 4, **caractérisé en ce que** lesdits moyens de fermeture inviolables comprennent en outre un élément annulaire (74) approprié pour interagir contre un percuteur spécifique (29) fourni sur la surface externe du corps formant récipient (2) au moyen d'une fente interne spécifique adaptée pour recevoir ledit percuteur (29), ledit élément annulaire (74) étant relié audit second capuchon (7a) au moyen d'un ou plusieurs éléments de liaison d'une manière sans correspondance.

6. Tube de test d'échantillonnage (1) selon la revendication précédente, **caractérisé en ce que** lesdits un ou plusieurs éléments de liaison sont constitués de languettes ou de pédoncules constitués du même matériau plastique que ledit second capuchon (7a) et ledit élément annulaire (74).

7. Tube de test d'échantillonnage (1) selon une ou plusieurs des revendications précédentes, **caractérisé en ce que** ledit second capuchon (7b) comprend une surface inférieure (70) dotée d'un trou traversant (73b).

8. Tube de test d'échantillonnage (1) selon la revendication 7, **caractérisé en ce que** ledit trou traversant (73b) est fermé par un film approprié pour être perforé par une sonde du type avec lequel un analyseur automatique ou un module de traitement automatique des étapes pré-analytiques est fourni.

9. Tube de test d'échantillonnage (1) selon la revendication 8, **caractérisé en ce que** ledit film est constitué d'aluminium-polyéthylène.

10. Tube de test d'échantillonnage (1) selon la revendication 7, **caractérisé en ce que** ledit second capuchon (7b) est soudé audit corps formant récipient (2).

11. Tube de test d'échantillonnage (1) selon la revendication 1, **caractérisé en ce que** ledit film est constitué d'aluminium-polyéthylène ou similaire.
